## Europäisches Patentamt

## European Patent Office

## Office européen des brevets

(11) Veröffentlichungsnummer: **0 121 782**
B1

(12)

# EUROPÄISCHE PATENTSCHRIFT

(45) Veröffentlichungstag der Patentschrift:
10.09.86

(21) Anmeldenummer: 84102499.5

(22) Anmeldetag: 08.03.84

(51) Int. Cl.⁴: **C 04 B 38/06**, C 03 C 11/00,
C 03 B 19/08, B 24 B 7/24,
B 24 C 1/06, C 03 C 15/00//
C03B29/00, A61L27/00,
B01J35/04

(54) Verfahren zur Herstellung poröser Überzüge oder poröser Körper aus Keramik, Glaskeramik oder Glas.

(30) Priorität: 16.03.83 DE 3309332

(43) Veröffentlichungstag der Anmeldung:
17.10.84 Patentblatt 84/42

(45) Bekanntmachung des Hinweises auf die Patenterteilung: 10.09.86 Patentblatt 86/37

(84) Benannte Vertragsstaaten:
AT CH DE FR GB IT LI NL

(56) Entgegenhaltungen:
AT-B- 251 755
DE-A- 2 759 214
DE-A- 3 202 623
DE-B- 2 453 882

(73) Patentinhaber: Pfaudler-Werke AG
Scheffelstrasse 55
D-6830 Schwetzingen (DE)

(72) Erfinder: Horsch, Rudi
Buchenweg 4
D-6831 Neulussheim (DE)

(74) Vertreter: Kosel, Peter, Dipl.-Ing. et al
Patentanwälte Dipl.-Inge. Röse, Kosel & Sobisch
Odastrasse 4a Postfach 129
D-3353 Bad Gandersheim 1 (DE)

Jouve, 18, rue St-Denis, 75001 Paris, France

**Beschreibung**

Die Erfindung betrifft ein Verfahren gemäß dem Oberbegriff des Anspruchs 1.

Bei einem bekannten Verfahren dieser Art (DE-Offenlegungsschrift 31 03 751) werden selbsttragende Körper mit offenen Poren und geringer Dichte in einem Sinterverfahren hergestellt. Dazu werden dem Pulver vor der Zugabe von Flüssigkeit organische Substanzen beigemischt. In einer auf die Vortrocknung folgenden ersten Aufheizstufe wird die organische Substanz zur Bildung der offenen Poren unter Gasbildung vollständig verbrannt, bevor sich eine zweite Temper- oder Sinterstufe anschließen kann. Nachteilig ist hierbei der Zeitverlust durch die zusätzliche erste Aufheizstufe. Oft sind ferner durchgehend offene Poren nicht erwünscht, z. B. dann, wenn unter einer derartigen Schicht korrosionsgefährdete Elemente liegen. Das durch die thermische Zersetzung der organischen Substanz entstehende Gas kann toxisch wirken, was insbesondere bei einer Verwendung bei Implantaten nicht tragbar ist. Die Verteilung und Größe der offenen Poren sind in unerwünschter Weise von der Korngröße der organischen Substanz und der Mischgüte abhängig.

Der Erfindung liegt die Aufgabe zugrunde, mit geringem Aufwand einen Überzug oder einen selbsttragenden Körper mit Poren zu schaffen, deren Verteilung sehr gleichmäßig und deren Größe beliebig einstellbar sind. Dabei sollen eine Veränderung der chemischen Zusammensetzung und eine Toxizität des Überzugs oder Körpers vermieden werden. Die Poren sollen grundsätzlich geschlossen sein, jedoch bei Bedarf, ggf. nur örtlich, geöffnet werden können.

Diese Aufgabe ist durch das kennzeichnende Merkmal des Anspruchs 1 gelöst. Durch Änderung der Temperatur und/oder der Zeitdauer der Lagerung des Gemisches kann die Menge des chemisch gebundenen Wassers und die Geschwindigkeit dieser chemischen Bindung nach Belieben beeinflußt werden. Das Temperaturniveau der Vortrocknung gemäß Schritt d) ist so niedrig, daß auf keinen Fall chemisch an das Pulver gebundenes Wasser vor dem Brennen gemäß Schritt e) von dem Pulver wieder abgespalten wird. Dies Abspaltung geschieht vielmehr erst während des Brennvorgangs und normalerweise bei Temperaturen oberhalb von 600 °C. Bei weichen Gläsern ist dies deren Erweichungsgebiet. Verwendet man derartige Gläser als Emailüberzug, entsteht während des Brennens durch das nach und nach abgespaltene, bis dahin chemisch gebundene Wasser und dessen Verdampfung eine Blasen- oder Porenstruktur in dem Überzug. Diese Poren sind grundsätzlich geschlossen und äußerst homogen in dem Überzug verteilt. Die Poren sind außerdem von günstig gleichmäßiger Größe, die sich vorzugsweise zwischen 20 und 500 μm steuern läßt. Bei Implantaten, mit denen der Interfacebereich eines Knochenlagers fest anwachsen soll, wird angesichts der Größe der in die Poren einwachsenden Knochenzellen eine Porengröße von 50 bis 150 μm angestrebt. Durch eine solche Porenstruktur wird das postoperative Anwachsen des Knochens an das Implantat verbessert und beschleunigt. Dazu werden vorzugsweise Poren an der Oberfläche des Implantats geöffnet, bevor das Implantat eingesetzt wird. Bei Implantaten können die geöffneten Poren außerdem als Depot für Zusatzstoffe, z. B. Medikamente, verwendet werden.

Überzüge oder selbsttragende Körper gemäß der Erfindung weisen nach einem Öffnen von Poren an der Oberfläche deutlich verbesserte Adhäsionseigenschaften bei Klebeverbindungen mit anderen Teilen auf. Die Erfindung eignet sich außerdem zur Herstellung von Katalysatoren für chemische Prozesse und die Variation der physikalischen Eigenschaften, z. B. der Wärmeleitfähigkeit, des Überzugs oder des selbsttragenden Körpers.

Der prozentuale Anteil der Poren in dem fertigen Überzug oder in dem fertigen selbsttragenden Körper kann durch die Höhe der Beladung des Pulvers mit chemisch gebundenem Wasser eingestellt werden.

Der Temperaturbereich gemäß Anspruch 2 ist bevorzugt. Versuchsüberzüge wurden mit sehr guten Ergebnissen aus einem Pulver gewonnen, das 8 h bei 140 °C gehalten wurde.

Die Lagerung in einem Autoklaven gemäß Anspruch 3 verkürzt erheblich die für eine ausreichende Beladung des Pulvers mit Wasser erforderliche Zeitdauer.

Gemäß Anspruch 4 läßt sich der prozentuale Anteil der Poren in dem fertigen Überzug oder dem fertigen selbsttragenden Körper auf einfache Weise einstellen.

Die Vortrocknung gemäß Anspruch 5 schafft gute Bedingungen für die Weiterverarbeitung und reproduzierbare Ergebnisse. Die Vortrocknung soll vorzugsweise verhältnismäßig vollständig erfolgen, so daß bei Beginn des Brennens gemäß Schritt e) nur noch eine minimale Feuchte in der vorgetrockneten Schicht oder dem vorgetrockneten Körper vorhanden ist. Durch übermäßige Restfeuchte könnte während des Brennens eine unerwünscht unkontrollierte Blasen- und damit Porenbildung erfolgen.

Die Merkmale des Anspruchs 6 oder 7 gestatten es, die Oberflächenporen in dem gewünschten Umfang zu den weiter vorne erwähnten Zwecken zu öffnen. Als Ätzmittel kann z. B. je nach der gewünschten Reaktionsgeschwindigkeit Flußsäure oder NaOH verwendet werden.

Das Merkmal des Anspruchs 8 erleichtert dem Ätzmedium das Eindringen in die geöffneten Poren auch bei verhältnismäßig feiner Blasenstruktur und damit ein Durchtrennen der Wände zwischen den Poren. So entstehen Verbindungskanäle zwischen benachbarten Poren. Durch diese Verbindungskanäle wird der interzellularen Flüssigkeit bei Verwendung als Implantat die Möglichkeit einer freien Zirkulation gegeben. Eine wachstumshemmende Ansammlung von pathologischen Stoffwechselprodukten wird vermieden, und die zur Erhaltung der Körperzellen erforderlichen biochemischen Substanzen können ungehindert transportiert werden.

Bei den Verfahren zum Öffnen von Poren gemäß den Ansprüchen 6 bis 8 entstehen mehr oder minder scharfkantige Ränder an den Poren. Die nachfolgende Wärmebehandlung gemäß Anspruch 9 beseitigt diese Scharfkantigkeit, wenn dies für den entsprechenden Einsatzfall gewünscht wird. Bei Emails liegt die Temperatur dieser nachfolgenden Wärmebehandlung z. B. zwischen 500 und 700 °C bei einer Behandlungsdauer zwischen 15 und 50 min. Diese Behandlungsdauer oder Brenndauer ist abhängig von der Größe und Geometrie des Werkstücks sowie von der Größe und dem prozentualen Anteil der Poren in dem Überzug oder dem selbsttragenden Körper.

## Beispiel

1 l Weißemailschlicker wird in einer verschlossenen PVC-Flasche 8 h auf 90 °C gehalten. Nach dem Abkühlen auf Raumtemperatur ist dieser Schlicker stark eingedickt, weil Wasser chemisch gebunden wurde. Durch Zugabe von Schlicker, dessen Pulver oder Fritte kein Wasser chemisch gebunden hat, z. B. im Verhältnis 50 : 50 %, und, falls gewünscht, durch Zugabe von Wasser, kann die Art der Blasenstruktur eingestellt und der Schlicker spritzfähig gemacht werden.

In ähnlicher Weise kann Emailschlicker hergestellt werden, der chemisch beständiger ist. Dabei wird die Wärmebehandlung vorzugsweise bei Temperaturen von z. B. 140 °C im Druckautoklaven durchgeführt.

Nach dem Trocknen und Einbrennen des beschichteten Werkstücks liegt zunächst ein zwar stark blasiger, aber geschlossener Überzug vor. Durch Ätzen mit 3 %iger Flußsäure während einer Zeitdauer von 5 min wird Email abgetragen und die Porenstruktur geöffnet. Die Flußsäurebehandlung erfolgt im Ultraschallbad, damit die Flußsäure besser in der Lage ist, in die feinen Poren einzudringen und die Seitenwände der Poren zu durchtrennen. Hierdurch entsteht in der so behandelten Oberflächenschicht ein Wabengerüst mit scharfen Kanten und von verhältnismäßig brüchiger Struktur.

Durch Nachbrennen bei 600 °C leicht oberhalb der Erweichungstemperatur bei einer Verweildauer von 30 min formen sich die scharfkantigen Ränder der geöffneten Poren zu gerundeten Strängen um. Dadurch wird die Festigkeit der geöffneten Poren wesentlich erhöht, die scharfen Kanten werden abgerundet, und es entsteht ein Emailüberzug mit spongiosaähnlicher Oberfläche, die als Implantat einer gegenüberliegenden Knochenfläche besonders günstige Bedingungen für das Ein- und Anwachsen bietet.

## Patentansprüche

1. Verfahren zur Herstellung eines porösen keramischen oder glaskeramischen oder glasigen Überzugs auf einem Gegenstand einerseits oder eines porösen keramischen oder glaskeramischen oder glasigen selbsttragenden Körpers andererseits, mit folgenden Schritten :

a) Eine keramische oder glaskeramische oder glasige Masse wird hergestellt und zu einem Pulver vermahlen,

b) das Pulver wird mit Wasser gemischt,

c) das Gemisch wird entweder in einer Schicht auf den mit dem Überzug zu versehenden Gegenstand aufgetragen oder zu dem Körper geformt,

d) die Schicht oder der Körper gemäß c) wird vorgetrocknet,

e) die vorgetrocknete Schicht oder der vorgetrocknete Körper gemäß d) wird gebrannt und gegebenenfalls einer nachfolgenden kontrollierten Wärmebehandlung zur Einstellung des Kristallanteils in dem Überzug oder in dem selbsttragenden Körper unterzogen, gekennzeichnet durch folgenden Schritt zwischen den Schritten b) und c) :

b1) das Gemisch gemäß b) wird bei einer Temperatur von 40 bis 200 °C während einer Zeitdauer gelagert, die es dem Pulver ermöglicht, Wasser chemisch zu binden.

2. Verfahren nach Anspruch 1, dadurch gekennzeichnet, daß die Lagerung gemäß b1) bei einer Temperatur von 100 bis 200 °C erfolgt.

3. Verfahren nach Anspruch 1 oder 2, dadurch gekennzeichnet, daß die Lagerung gemäß b1) in einen Autoklaven geschieht.

4. Verfahren nach einem der Ansprüche 1 bis 3, dadurch gekennzeichnet, daß das gemäß b1) hergestellte Gemisch mit dem wasserbeladenen Pulver vor dem Schritt c) mit nicht wasserbeladenem Pulver gemäß a) gemischt wird.

5. Verfahren nach einem der Ansprüche 1 bis 4, dadurch gekennzeichnet, daß die Vertrocknung gemäß d) zwischen Raumtemperatur und 80 °C durchgeführt wird.

6. Verfahren nach einem der Ansprüche 1 bis 5, dadurch gekennzeichnet, daß im Anschluß an e) die Oberfläche des Überzugs oder des selbsttragenden Körpers zum Öffnen von im Bereich dieser Oberfläche befindlichen geschlossenen Poren mechanisch bearbeitet, z. B. überschliffen oder sandgestrahlt, wird.

7. Verfahren nach einem der Ansprüche 1 bis 5, dadurch gekennzeichnet, daß im Anschluß an e) die Oberfläche des Überzugs oder des selbsttragenden Körpers zum Öffnen von im Bereich dieser Oberfläche befindlichen geschlossenen Poren durch Ätzung abgetragen wird.

8. Verfahren nach Anspruch 7, dadurch gekennzeichnet, daß die Ätzung in einem Ultraschallbad geschieht.

9. Verfahren nach einem der Ansprüche 6 bis 8, gekennzeichnet durch eine nachfolgende Wärmebehandlung bei einer Temperatur wenig oberhalb der Erweichungstemperatur der Oberfläche bis zum Rundschmelzen scharfkantiger Ränder der geöffneten Poren.

## Claims

1. Method for preparing a porous ceramic or glass-ceramic or glassy coating on an article, on the one hand, or a porous ceramic or glass-ceramic or glassy self-supporting body, on the other hand, including the following steps:

(a) a ceramic or glass-ceramic or glassy mass is made and ground to a powder,

(b) the powder is mixed with water,

(c) the mixture is either applied in a layer to the article to be provided with the coating or is formed into the body,

(d) the layer or the body according to (c) is pre-dried,

(e) the pre-dried layer or the pre-dried body according to (d) is fired and, if required, is then subjected to a controlled heat treatment for adjusting the crystal fraction formed in the coating or in the self-supporting body, characterized by the following step between steps (b) and (c):

(b1) the mixture according to (b) is maintained at a temperature from 40° to 200 °C for a time period which enables water to bind chemically with the powder.

2. Method according to claim 1, characterized in that the treatment according to (b1) takes place at a temperature from 100° to 200 °C.

3. Method according to claim 1 or 2, characterized in that the treatment according to (b1) occurs in an autoclave.

4. Method according to any of claims 1 to 3, characterized in that the mixture made with the water-laden powder according to (b1) is mixed before step (c) with non-water-laden powder according to (a).

5. Method according to any of claims 1 to 4, characterized in that the pre-drying according to (d) is carried out between room temperature and 80 °C.

6. Method according to any of claims 1 to 5, characterized in that, following (e), the surface of the layer or the self-supporting body is mechanically treated, e.g. ground or sand-blasted, to open closed pores located in the region of such surface.

7. Method according to any of claims 1 to 5, characterized in that, following (e), the surface of the coating or the self-supporting body is subjected to etching to open closed pores located in the region of such surface.

8. Method according to claim 7, characterized in that the etching is carried out in an ultra-sound bath.

9. Method according to any of claims 6 to 8, characterized by a subsequent heat treatment at a temperature slightly above the softening temperature of the surface so as to round off by metting sharp-edged portions of the opened pores.

## Revendications

1. Procédé pour la production d'un revêtement poreux céramique ou vitrocéramique ou vitreux sur un objet d'une part, ou d'un corps autoporteur poreux céramique ou vitrocéramique ou vitreux d'autre part, comportant les stades suivants:

a) une masse céramique ou vitrocéramique ou vitreuse est fabriquée et broyée pour fournir une poudre;

b) la poudre est mélangée avec de l'eau;

c) le mélange est, soit appliqué en couche sur l'objet à pourvoir du revêtement, soit mis en forme pour constituer le corps;

d) la couche ou le corps selon c) sont préséchés;

e) la couche préséchée, ou le corps préséché, selon d) est cuit et éventuellement soumis à un traitement thermique contrôlé consécutif pour régler la proportion de cristaux dans le revêtement ou dans le corps autoporteur caractérisé par le stade suivant s'insérant entre les stades b) et c):

b1) le mélange selon b) est maintenu à une température de 40 à 200 °C, pendant un temps qui permet à la poudre de lier chimiquement de l'eau.

2. Procédé selon la revendication 1, caractérisé en ce que le maintien selon b1) s'effectue à une température de 100 à 200 °C.

3. Procédé selon la revendication 1 ou 2, caractérisé en ce que le maintien selon b1) s'effectue dans un autoclave.

4. Procédé selon l'une des revendications 1 à 3, caractérisé en ce que le mélange produit selon b1) avec la poudre chargée d'eau est mélangé avant le stade c) avec de la poudre non chargée d'eau selon a).

5. Procédé selon l'une des revendications 1 à 4, caractérisé en ce que le préséchage selon d) est effectué à une température entre la température ambiante et 80 °C.

6. Procédé selon l'une des revendications 1 à 5, caractérisé en ce que consécutivement à e) la surface du revêtement ou du corps autoporteur est usinée mécaniquement pour ouvrir des pores fermés se trouvant dans la région de cette surface, par exemple rabotée ou sablée au jet.

7. Procédé selon l'une des revendications 1 à 5, caractérisé en ce que consécutivement à e) la surface du revêtement ou du corps autoporteur est réduite par attaque chimique pour ouvrir des pores fermés se trouvant dans la région de cette surface.

8. Procédé selon la revendication 7, caractérisé en ce que l'attaque chimique s'effectue dans un bain à ultra-sons.

9. Procédé selon l'une des revendications 6 à 8, caractérisé en ce qu'il comprend un traitement thermique consécutif à une température légèrement supérieure à la température de ramollissement de la surface jusqu'à ce que les bords aigus des pores ouverts soient arrondis par fusion.